# EUROPÄISCHE PATENTSCHRIFT

(11) **EP 1 641 414 B1**
(45) Veröffentlichungstag und Bekanntmachung des Hinweises auf die Patenterteilung: **24.10.2007**
(21) Anmeldenummer: 04740454.6
(22) Anmeldetag: 30.06.2004
(51) Int. Cl.: A61F 2/46, A61F 2/44

(54) **CHIRURGISCHES INSTRUMENT ZUM HANDHABEN EINES IMPLANTATS**
SURGICAL INSTRUMENT FOR HANDLING AN IMPLANT
INSTRUMENT CHIRURGICAL SERVANT A MANIPULER UN IMPLANT

(30) Priorität: 08.07.2003 DE 10330699
(43) Veröffentlichungstag der Anmeldung: 05.04.2006
(73) Patentinhaber: Aesculap AG & Co. KG, 78532 Tuttlingen (DE)
(72) Erfinder: SCHULTZ, Robert, 78532 Tuttlingen (DE)
(74) Vertreter: Boehme, Ulrich
(86) Internationale Anmeldenummer: PCT/EP2004/007072
(87) Internationale Veröffentlichungsnummer: WO 2005/004757

(56) Entgegenhaltungen:
- WO-A-01/19295
- US-A- 5 314 477
- US-B1- 6 540 785

## Beschreibung

Die Erfindung betrifft ein chirurgisches Instrument mit einer Halteeinrichtung am Instrument und mit an der Halteeinrichtung gehaltenen Teilen eines zwei Teile umfassenden Implantats, die jeweils mit einer dem anderen Teil zugewandten Gelenkfläche versehen sind, die im implantierten Zustand aneinander anliegen und dadurch eine Verschwenkung der beiden Teile gegeneinander ermöglichen, und die durch die Halteeinrichtung lösbar mit dem Instrument verbunden sind, wobei am Instrument ein Distanzelement angeordnet ist, welches zwischen die Teile eingreift.

Ein Handhabungsinstrument für ein dreiteiliges chirurgisches Implantat ist aus der WO 01/19295 A1 bekannt und dient dazu, ein Zwischenwirbelimplantat aufzunehmen und dann in den Zwischenwirbelraum zwischen zwei Wirbelkörpern einzuführen. Die beiden Teile des Implantats werden bei dem bekannten Instrument dicht aneinanderliegend in den Zwischenwirbelraum eingeführt und dann dort so weit voneinander entfernt, daß zwischen sie ein Einsatz eingeschoben werden kann, der die Gelenkfläche trägt.

In der US 6,540,785 A ist ein Handhabungsinstrument für ein zweiteiliges Implantat beschrieben, bei dem die Implantatteile so an dem Handhabungsinstrument festgelegt sind, dass die Gelenkflächen der beiden Implantatteile einander berühren.

Bei der Verwendung von zweiteiligen Implantaten, bei denen die beiden Teile jeweils eine Gelenkfläche tragen, die im eingesetzten Zustand flächig aneinander anliegen und dadurch eine Verschwenkbarkeit der beiden Teile ermöglichen, werden die beiden Teile zusammen in den Zwischenwirbelraum eingeführt, und dabei besteht die Gefahr, daß die Gelenkflächen beschädigt werden können. Diese Gefahr ist insbesondere dann groß, wenn die Gelenkflächen empfindlich sind, beispielsweise aus Keramik bestehen, und daher Stöße schlecht vertragen können. Beim Implantieren des Implantates ist aber nicht zu vermeiden, daß derartige Stöße auf das Implantat ausgeübt werden, beispielsweise beim Einschlagen des Implantates in den Zwischenwirbelraum.

Es ist Aufgabe der Erfindung, ein gattungsgemäßes chirurgisches Instrument so auszubilden, daß die Gefahr einer Beschädigung der Gelenkflächen des Implantates beim Handhaben des Implantates herabgesetzt wird.

Diese Aufgabe wird bei einem chirurgischen Instrument der eingangs beschriebenen Art erfindungsgemäß dadurch gelöst, daß das Distanzelement (8) die Teile (21, 22) in einem so großen Abstand voneinander hält, daß die Gelenkflächen (27, 28) der beiden Teile (21, 22) des Implantats (20) sich nicht berühren.

Die beiden Teile werden also so weit voneinander entfernt, daß beim Handhaben und insbesondere beim Einsetzen in den Zwischenwirbelraum keinerlei Berührung zwischen den Gelenkflächen auftreten kann, so daß durch Stöße oder dergleichen auch die Gefahr einer Beschädigung dieser Gelenkflächen minimiert wird.

Dabei ist es vorteilhaft, wenn der Abstand der Gelenkflächen bei zwischen die Teile des Implantats eingreifendem Distanzelement zwischen 0,2 und 2 Millimetern liegt, also sehr klein ist, so daß die gesamte Bauhöhe des Implantates zum Einführen nur unwesentlich gegenüber der Bauhöhe erhöht wird, die das Implantat im funktionsfähigen Zustand aufweist, wenn also die Gelenkflächen aneinander anliegen.

Das Distanzelement kann insbesondere plattenförmig ausgebildet sein.

Es ist günstig, wenn die den Teilen zugewandte Oberseite und die Unterseite des Distanzelementes eben ausgebildet sind, vorzugsweise weist dann auch das Implantat entsprechende ebene Flächen auf, so daß eine flächige Anlage erfolgt, die das Auftreten von Druckspitzen minimiert.

Bei einer besonders bevorzugten Ausführungsform ist vorgesehen, daß die den Teilen zugewandte Oberseite und die Unterseite des Distanzelementes geringfügig gegeneinander geneigt sind, das Distanzelement hat also eine geringe Keilform, dadurch wird das Herausziehen des Distanzelementes nach der Implantatation des Implantates erleichtert.

Das Distanzelement kann einstückig ausgebildet sein, bei einer bevorzugten Ausführungsform ist jedoch vorgesehen, daß das Distanzelement zwei die Gelenkflächen zwischen sich aufnehmende Distanzglieder umfaßt. Diese Distanzglieder liegen auf gegenüberliegenden Seiten der Gelenkflächen, beispielsweise eines die Gelenkflächen ausbildenden Einsatzes in die Teile des Implantates, so daß die Teile des Implantates an beiden Seiten symmetrisch auf Abstand gehalten werden. Beispielsweise können die Distanzglieder parallel zueinander verlaufen, sie haben dann Ähnlichkeit mit den Zinken einer zweizinkigen Gabel.

Bei einer besonders bevorzugten Ausführungsform ist vorgesehen, daß die Halteeinrichtung und die Teile des Implantates zur lösbaren Verbindung ineinander schiebbare Klemmelemente aufweisen, die quer zu ihrer Einschubrichtung gegeneinander verspannbar sind. Beim Einschieben sind diese Klemmelemente noch nicht verspannt, das Einschieben und das Herausziehen ist dadurch einfach möglich. Durch die Verspannung werden jedoch die Klemmelemente so verklemmt, daß ein Abziehen der Teile des Implantates von der Halteeinrichtung stark erschwert oder gar unmöglich ist.

Die Klemmelemente können insbesondere ineinander greifende Vor- und Rücksprünge sein.

Bei einer bevorzugten Ausführungsform sind die Vor- und Rücksprünge Stifte und diese aufnehmende Bohrungen.

Es ist dabei vorteilhaft, wenn die Klemmelemente in einer Richtung gegeneinander verspannbar sind, die quer zur Verschiebung der Teile des Implantates durch das zwischen sie eingreifende Distanzelement verläuft. Durch die Spannung der Klemmelemente werden damit nicht die Teile des Implantates gegen das Distanzelement gespannt, sondern die Andruckkräfte der Teile des Implantates gegen das Distanzelement sind davon unabhängig, ob die Klemmelemente gespannt sind oder nicht.

Die Klemmelemente können bei einer bevorzugten Ausführungsform der Erfindung am Instrument angeordnet sein und quer zur Einschubrichtung der Klemmelemente verschiebbar sein.

Beispielsweise kann bei einer bevorzugten Ausführungsform vorgesehen sein, daß das Instrument zwei gegeneinander schwenkbare Arme aufweist, die jeweils an ihrem freien Ende Klemmelemente für die beiden Teile tragen, und daß am Instrument eine Spannvorrichtung angeordnet ist, mit der die Arme gegeneinander schwenkbar sind. Die Arme können federnd gegeneinander schwenkbar sein.

Die Spannvorrichtung wird bei einer bevorzugten Ausführungsform durch eine die Arme übergreifende und längs der Arme bewegbare Hülse gebildet. Diese ist zur Bewegung längs der Arme vorzugsweise um ihre Längsachse verdrehbar und über ein Schraubgewinde an dem Instrument gelagert. Durch Verdrehen der Hülse wird diese längs des Armes verschoben und spannt dabei die beiden Arme gegeneinander.

Jeder der beiden Arme kann ein Distanzglied tragen.

Bei einer besonders bevorzugten Ausführungsform ist vorgesehen, daß jeder Arm an seinem freien Ende einen Klemmbacken trägt, an dem jeweils Klemmelemente für beide Teile des Implantates und ein Distanzglied angeordnet sind und die einen Anschlag bilden, an dem die Teile des Implantates anliegen, wenn das Instrument und das Implantat verbunden sind.

Die nachfolgende Beschreibung bevorzugter Ausführungsformen der Erfindung dient im Zusammenhang mit der Zeichnung der näheren Erläuterung. Es zeigen:

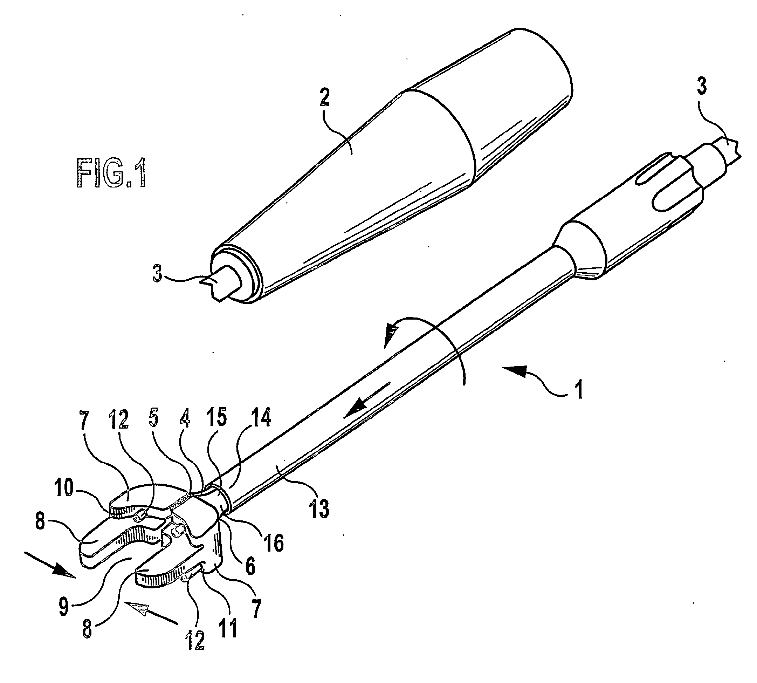

- Figur 1:: eine perspektivische Ansicht eines chirurgischen Instruments zur Handhabung eines Implantates;

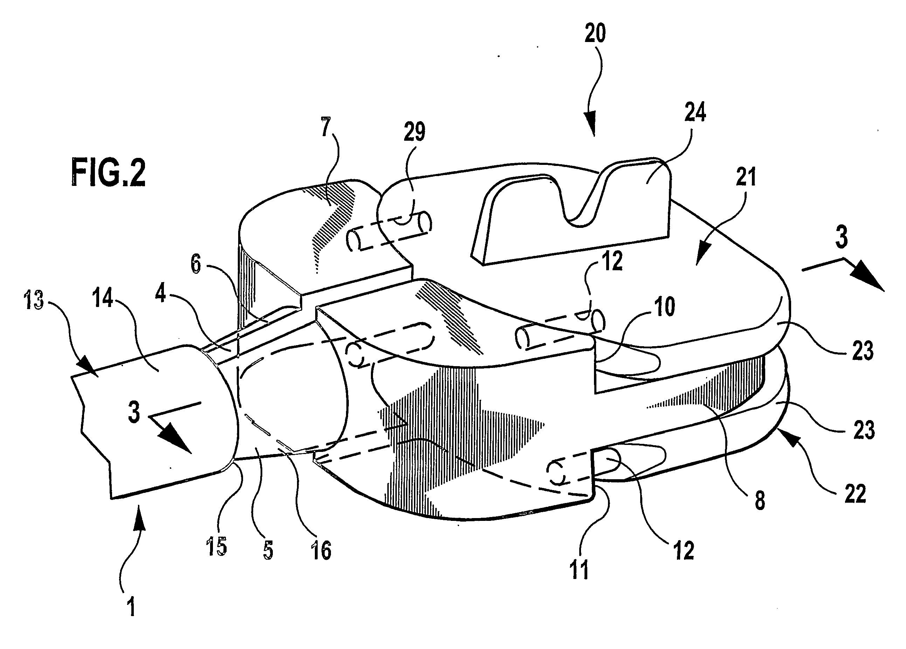

- Figur 2:: eine vergrößerte Detailansicht des distalen Endes des chirurgischen Instrumentes der Figur 1 mit einem an ihm gehaltenen zweiteiligen Zwischenwirbelimplantat und

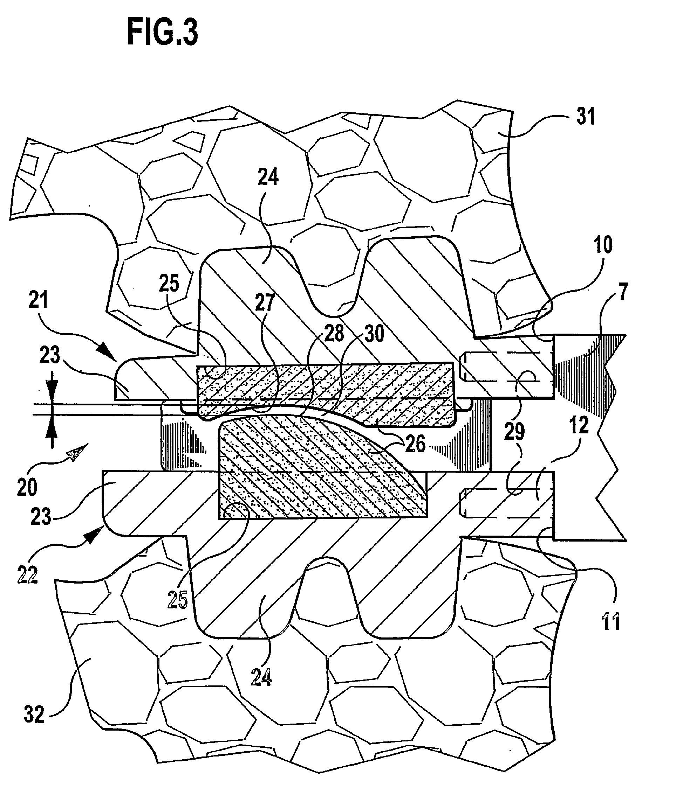

- Figur 3:: eine Schnittansicht längs Linie 3-3 in Figur 2.

Das in der Zeichnung dargestellte chirurgische Instrument 1 umfaßt einen Handgriff 2 mit einem daran gehaltenen Stab 3, der an seinem dem Handgriff 2 gegenüberliegenden Ende durch einen Längsschlitz 4 in zwei parallele Arme 5, 6 unterteilt ist, die federnd gegeneinander oder auseinander geschwenkt werden können. Beide Arme sind gleich ausgebildet, nachstehend wird daher nur jeweils ein Arm ausführlich beschrieben. Am Ende des Armes trägt dieser einen Klemmbacken 7, der einstückig mit dem Arm ausgebildet sein kann und breiter und höher ist als dieser. An jedem Klemmbacken 7 ist ein im wesentlichen plattenförmiges Distanzglied 8 angeordnet, dieses erstreckt sich in Längsrichtung des Stabes 3 auf der dem Handgriff 2 abgewandten Seite des Klemmbackens 7, die beiden Distanzglieder 8 der beiden Klemmbacken 7 verlaufen in Richtung des Stabes 3 parallel und im Abstand zueinander, so daß zwischen den plattenförmigen Distanzgliedern 8 ein Zwischenraum 9 freibleibt. Die Distanzglieder 8 können als Platten mit paralleler, ebener Ober- und Unterseite ausgebildet sein, es ist aber auch möglich, daß sie zum freien Ende hin eine etwas geringere Dicke aufweisen, also geringfügig keilförmig ausgebildet sind.

Die Dicke des Distanzgliedes 8 ist geringer als die Dicke der Klemmbacken 7, im Übergangsbereich bilden die Klemmbacken 7 auf der Oberseite und auf der Unterseite des Distanzgliedes 8 je eine Stufe 10, 11 aus, und im Bereich dieser Stufen 10, 11 stehen vom Klemmbacken 7 parallel zum Stab 3 verlaufende Stifte 12 ab. Dabei trägt jeder Klemmbacken 7 jeweils oberhalb und unterhalb des Distanzgliedes 8 einen derartigen Stift 12, das Instrument 1 weist also insgesamt vier derartige Stifte 12 auf, die parallel zueinander verlaufen und wesentlich kürzer sind als die Distanzglieder 8.

Auf dem Stab 3 ist eine diesen umgebende Hülse 13 um die Stablängsachse drehbar gelagert, die mit ihrem dem Handgriff 2 zugewandten Ende 14 auf ein Gewinde des Stabes 3 aufgeschraubt ist, so daß sie beim Verschrauben auf diesem Gewinde in Längsrichtung des Stabes 3 bewegt wird. Die Hülse 13 umgibt mit ihrem dem Handgriff 2 abgewandten distalen Ende die beiden Arme 5, 6 in dem sich unmittelbar an die Klemmbacken 7 anschließenden Abschnitt 15, und in diesem Abschnitt 15 sind die Arme 5, 6 seitlich so verbreitert, daß sie auf beiden Seiten schräg verlaufende Anlaufflächen 16 ausbilden. Wenn die Hülse 13 durch Verdrehung auf dem Gewinde in Richtung auf die Klemmbacken 7 vorgeschoben wird, gleitet sie an diesen Anlaufflächen 16 entlang und verschwenkt dadurch die Arme 5, 6 gegeneinander, d.h. der Längsschlitz 4 wird dadurch schmaler.

Das beschriebene Instrument 1 dient der Aufnahme eines Implantates 20, welches zwei Teile 21, 22 umfaßt. Beide Teile weisen einen ebenen, plattenförmigen Träger 23 auf, der an seiner Außenseite Verankerungsvorsprünge 24 trägt, während auf der Innenseite in eine Vertiefung 25 ein Keramikeinsatz 26 eingesetzt ist. Während die Träger im wesentlichen gleich aufgebaut sind, weisen die Keramikeinsätze 26 zusammen wirkende Gelenkflächen 27, 28 auf, die im wesentlichen Kugelteilflächen sind, eine Gelenkfläche 27 ist dabei konkav ausgebildet, die andere konvex, beide Gelenkflächen 27 und 28 sind komplementär zueinander und ermöglichen bei einer flächigen Anlage aneinander eine verschwenkbare Abstützung der beiden Teile 21, 22 gegeneinander.

Beide Träger 23 weisen jeweils zwei parallel zueinander verlaufende Sacklochbohrungen 29 auf, die der Aufnahme der Stifte 12 des Instrumentes 1 dienen. Der gegenseitige Abstand der Sacklochbohrungen 29 an einem Träger 23 entspricht dem gegenseitigen Abstand der Stifte 12 an nebeneinanderliegenden Klemmbacken 7 bei unverschwenkten Armen 5, 6, so daß die Stifte 12 vor dem Vorschieben der Hülse 13 leicht in die entsprechenden Sacklochbohrungen 29 der Träger 23 eingeschoben werden können. Die Träger 23 werden dabei auf den Stiften gehalten und legen sich an die Stufen 10 der Klemmbacken 7 an, die damit die Eintauchtiefe der Stifte 12 begrenzen.

Die Abmessungen und Anordnungen der Stifte 12, der Sacklochbohrungen 29 sowie der Distanzglieder 8 sind so gewählt, daß nach dem Aufschieben der Träger 23 auf die Stifte 12 diese geringfügig im Abstand zueinander gehalten werden, so daß die Gelenkflächen 27, 28 sich nicht berühren, sondern einen kleinen Abstand haben, der Spalt 30 zwischen den Gelenkflächen 27 und 28 ist dabei nicht breit, die Breite liegt beispielsweise zwischen 0,2 Millimetern und 2 Millimetern. Dieser Abstand zwischen den Gelenkflächen 27 und 28 wird insbesondere dadurch erzeugt und eingehalten, daß die Distanzglieder 8 zwischen die beiden Träger 23 eingreifen und die Träger 23 mit ihren einander zugewandten Innenflächen auf diesen Distanzgliedern 8 aufliegen, so daß eine weitere Annäherung der Gelenkflächen 27, 28 auch bei mechanischer Beanspruchung der Träger 23 mit Sicherheit vermieden wird. Dabei liegen die Distanzglieder 8 auf gegenüberliegenden Seiten der Gelenkflächen 27 und 28 und der Keramikeinsätze 26, so daß die Keramikeinsätze 26 zwischen den beiden Distanzgliedern 8 schützend aufgenommen sind.

Die Träger 23 können in diesem auf die Arme 8 aufgeschobenen Zustand dadurch fixiert werden, daß die Hülse 13 durch Verschraubung in Richtung auf die Klemmbacken 7 vorgeschoben wird, dabei legt sie sich an die Anlaufflächen 16 an und verschwenkt die Arme 5, 6 aufeinander zu. Dadurch werden die Stifte 12 in den Sacklochbohrungen 29 gegeneinander gespannt, es ergibt sich eine Verklemmung und dadurch ein sicherer Sitz der Träger 23 an den Klemmbacken 7.

Wenn das Implantat in dieser Weise am Instrument festgelegt ist, kann das Implantat mittels des Instrumentes 1 sicher und zuverlässig gehandhabt werden, insbesondere kann auf diese Weise das Implantat in einen Zwischenwirbelraum zwischen zwei Wirbel körpern 31, 32 eingeschoben werden, das Einschieben kann durch Hammerschläge unterstützt werden, die auf die Rückseite des Handgriffes 2 ausgeführt werden. Dabei ist sichergestellt, daß die Schlagkräfte nicht zu einer Beschädigung der Gelenkflächen 27, 28 führen können.

Nach dem Einsetzen kann durch Zurückschrauben der Hülse 13 die Verklemmung der Träger 23 an den Klemmbacken 7 wieder gelöst werden, und dann kann das Instrument 1 vom Implantat abgezogen werden. Eine keilförmige Ausbildung der Distanzglieder 8 erleichtert ein solches Herausziehen, außerdem kann durch diese Keilform gegebenenfalls eine Anpassung an die Geometrie der Träger 23 erfolgen, deren Innenflächen nicht unbedingt parallel zueinander stehen müssen, sondern gegebenenfalls auch einen kleinen Winkel miteinander einschließen können. Beim Herausziehen der Distanzglieder 8 aus dem Zwischenraum zwischen den Trägern 23 werden diese so weit einander angenähert, daß die Gelenkflächen 27, 28 zur Anlage aneinander kommen und somit über die Träger 23 die benachbarten Wirbelkörper 31, 32 gelenkig gegeneinander abstützen.

## Patentansprüche

1. Chirurgisches Instrument (1) mit einer Halteeinrichtung (5, 6) am Instrument (1) und mit an der Halteeinrichtung (5, 6) gehaltenen Teilen (21, 22) eines zwei Teile umfassenden Implantats (20), die jeweils mit einer dem anderen Teil zugewandten Gelenkfläche (27, 28) versehen sind, die im implantierten Zustand aneinander anliegen und **dadurch** eine Verschwenkung der beiden Teile (21, 22) gegeneinander ermöglichen, und die durch die Halteeinrichtung (5, 6) lösbar mit dem Instrument (1) verbunden sind, wobei am Instrument (1) ein Distanzelement (8) angeordnet ist, welches zwischen die Teile (21, 22) eingreift, **dadurch gekennzeichnet, dass** das Distanzelement (8) die Teile (21, 22) in einem so großen Abstand voneinander hält, daß die Gelenkflächen (27, 28) der beiden Teile (21, 22) des Implantats (20) sich nicht berühren.

2. Instrument nach Anspruch 1, **dadurch gekennzeichnet, daß** der Abstand der Gelenkflächen (27, 28) bei zwischen die Teile (21, 22) des Implantats (20) eingreifendem Distanzelement (8) zwischen 0,2 und 2 Millimetern liegt.

3. Instrument nach einem der voranstehenden Ansprüche, **dadurch gekennzeichnet, daß** das Distanzelement (8) plattenförmig ausgebildet ist.

4. Instrument nach einem der voranstehenden Ansprüche, **dadurch gekennzeichnet, daß** die den Teilen (21, 22) des Implantats (20) zugewandte Oberseite und Unterseite des Distanzelementes (8) eben ausgebildet sind.

5. Instrument nach einem der voranstehenden Ansprüche, **dadurch gekennzeichnet, daß** die den Teilen (21, 22) des Implantats (20) zugewandte Oberseite und Unterseite des Distanzelementes (8) geringfügig gegeneinander geneigt sind.

6. Instrument nach einem der voranstehenden Ansprüche, **dadurch gekennzeichnet, daß** das Distanzelement zwei die Gelenkflächen (27, 28) zwischen sich aufnehmende Distanzglieder (8) umfaßt.

7. Instrument nach Anspruch 6, **dadurch gekennzeichnet, daß** die Distanzglieder parallel zueinander verlaufen.

8. Instrument nach einem der voranstehenden Ansprüche, **dadurch gekennzeichnet, daß** die Halteeinrichtung (5, 6) und die Teile (21, 22) des Implantats (20) zur lösbaren Verbindung ineinander schiebbare Klemmelemente (12; 29) aufweisen, die quer zu ihrer Einschubrichtung gegeneinander verspannbar sind.

9. Instrument nach Anspruch 8, **dadurch gekennzeichnet, daß** die Klemmelemente (12; 29) ineinander greifende Vor- und Rücksprünge sind.

10. Instrument nach Anspruch 9, **dadurch gekennzeichnet, daß** die Vor- und Rücksprünge Stifte (12) und diese aufnehmende Bohrungen (29) sind.

11. Instrument nach Anspruch 8 oder 9, **dadurch gekennzeichnet, daß** die Klemmelemente (12, 29) in einer Richtung gegeneinander verspannbar sind, die quer zur Verschiebung der Teile (21, 22) des Implantats (20) durch das zwischen sie eingreifende Distanzelement (8) verläuft.

12. Instrument nach einem der Ansprüche 8 bis 11, **dadurch gekennzeichnet, daß** die Klemmelemente (12) am Instrument (1) quer zu ihrer Einschubrichtung verschiebbar sind.

13. Instrument nach Anspruch 12, **dadurch gekennzeichnet, daß** das Instrument (1) zwei gegeneinander schwenkbare Arme (5, 6) aufweist, die jeweils an ihrem freien Ende Klemmelemente (12) für die beiden Teile (21, 22) des Implantats (20) tragen, und daß am Instrument (1) eine Spannvorrichtung (13) angeordnet ist, mit der die Arme (5, 6) gegeneinander schwenkbar sind.

14. Instrument nach Anspruch 13, **dadurch gekennzeichnet, daß** die Arme (5, 6) federnd gegeneinander schwenkbar sind.

15. Instrument nach Anspruch 14, **dadurch gekennzeichnet, daß** die Spannvorrichtung eine die Arme (5, 6) übergreifende und längs der Arme (5, 6) bewegbare Hülse (13) ist.

16. Instrument nach Anspruch 15, **dadurch gekennzeichnet, daß** die Hülse (13) zur Bewegung längs der Arme (5, 6) um ihre Längsachse verdrehbar und über ein Schraubgewinde an dem Instrument (1) gelagert ist.

17. Instrument nach einem der Ansprüche 13 bis 16, **dadurch gekennzeichnet, daß** jeder Arm (5, 6) ein Distanzglied (8) trägt.

18. Instrument nach einem der Ansprüche 13 bis 17, **dadurch gekennzeichnet, daß** jeder Arm (5, 6) an seinem freien Ende einen Klemmbacken (7) trägt, an dem jeweils Klemmelemente (12) für beide Teile (21, 22) des Implantats (20) und ein Distanzglied (8) angeordnet sind und die einen Anschlag (10) bilden, an dem die Teile (21, 22) des Implantats (20) anliegen, wenn das Instrument (1) und das Implantat (20) verbunden sind.

## Claims

1. Surgical instrument (1), with a holding device (5, 6) on the instrument (1), and with parts (21, 22), held on the holding device (5, 6), of an implant (20) having two parts, each of which is provided with a joint surface (27, 28) facing the other part, the joint surfaces being in contact with each other in the implanted state and thereby permitting a pivoting of the two parts (21, 22) in relation to each other, and the parts being detachably connected to the instrument (1) by the holding device (5, 6), and a spacer element (8) being arranged on the instrument (1) and extending between the parts (21, 22), **characterized in that** the spacer element (8) keeps the parts (21, 22) at such a large distance from each other that the joint surfaces (27, 28) of the two parts (21, 22) of the implant (20) do not contact each other.

2. Instrument in accordance with claim 1, **characterized in that** the distance between the joint surfaces (27, 28) is between 0.2 mm and 2 mm when the spacer element (8) extends between the parts (21, 22) of the implant (20).

3. Instrument in accordance with any one of the preceding claims, **characterized in that** the spacer element (8) is plate-shaped.

4. Instrument in accordance with any one of the preceding claims, **characterized in that** the top side and the underside of the spacer element (8) that face the parts (21, 22) of the implant (20) are flat.

5. Instrument in accordance with any one of the preceding claims, **characterized in that** the top side and the underside of the spacer element (8) that face the parts (21, 22) of the implant (20) are slightly sloped in relation to each other.

6. Instrument in accordance with any one of the preceding claims, **characterized in that** the spacer element comprises two spacer members (8) for receiving the joint surfaces (27, 28) between them.

7. Instrument in accordance with claim 6, **characterized in that** the spacer members extend parallel to each other.

8. Instrument in accordance with any one of the preceding claims, **characterized in that** the holding device (5, 6) and the parts (21, 22) of the implant (20) comprise clamping elements (12; 29) configured to be pushed into each other for a detachable connection and adapted to be braced against each other at right angles to the direction in which they are pushed in.

9. Instrument in accordance with claim 8, **characterized in that** the clamping elements (12; 29) are projections and recesses engaging each other.

10. Instrument in accordance with claim 9, **characterized in that** the projections and recesses are pins (12) and holes (29) receiving the latter.

11. Instrument in accordance with claim 8 or 9, **characterized in that** the clamping elements (12, 29) are adapted to be braced against each other in a direction that extends at right angles to the displacement of the parts (21, 22) of the implant (20) by the spacer element (8) extending between them.

12. Instrument in accordance with any one of claims 8 to 11, **characterized in that** the clamping elements (12) are displaceable on the instrument (1) at right angles to the direction in which they are pushed in.

13. Instrument in accordance with claim 12, **characterized in that** the instrument (1) comprises two arms (5, 6) which are pivotable in relation to each other and each carry at their free end clamping elements (12) for the two parts (21, 22) of the implant (20), and **in that** a tensioning device (13), with which the arms (5, 6) are pivotable in relation to each other, is arranged on the instrument (1).

14. Instrument in accordance with claim 13, **characterized in that** the arms (5, 6) are elastically pivotable in relation to each other.

15. Instrument in accordance with claim 14, **characterized in that** the tensioning device is a sleeve (13) which extends over the arms (5, 6) and is movable along the arms (5, 6).

16. Instrument in accordance with claim 15, **characterized in that** the sleeve (13) is rotatable around its longitudinal axis for movement along the arms (5, 6) and is mounted on the instrument (1) by means of a screw thread.

17. Instrument in accordance with any one of claims 13 to 16, **characterized in that** each arm (5, 6) carries a spacer member (8).

18. Instrument in accordance with any one of claims 13 to 17, **characterized in that** each arm (5, 6) carries at its free end a clamping jaw (7), on each of which clamping elements (12) for both parts (21, 22) of the implant (20) and a spacer member (8) are arranged, and each of which forms a stop (10), with which the parts (21, 22) of the implant (20) are in contact when the instrument (1) and the implant (20) are connected.

## Revendications

1. Instrument chirurgical (1) comportant un dispositif de retenue (5, 6) sur l'instrument (1) et des parties (21, 22) retenues sur le dispositif de retenue (5, 6), d'un implant (20) comprenant deux parties, qui sont chacune pourvues d'une surface d'articulation (27, 28) tournée vers l'autre partie et qui, à l'état implanté, sont en appui l'une sur l'autre et permettent ainsi un pivotement des deux parties (21, 22) l'une par rapport à l'autre, et qui sont reliées de manière détachable à l'instrument (1) par le dispositif de retenue (5, 6), un élément d'espacement (8) étant agencé sur l'instrument (1), lequel s'engage entre les parties (21, 22), **caractérisé en ce que** l'élément d'espacement (8) maintient les pièces (21, 22) à une distance si grande l'une de l'autre que les surfaces d'articulation (27, 28) des deux parties (21, 22) de l'implant (20) ne se touchent pas.

2. Instrument selon la revendication 1, **caractérisé en ce que** la distance des surfaces d'articulation (27, 28) est entre 0,2 et 2 mm lorsque l'élément d'espacement (8) s'engage entre les parties (21, 22) de l'implant (20).

3. Instrument selon l'une des revendications précédentes, **caractérisé en ce que** l'élément d'espacement (8) est réalisé en forme de plaque.

4. Instrument selon l'une des revendications précédentes, **caractérisé en ce que** la face supérieure et la face inférieure de l'élément d'espacement (8), tournées vers les parties (21, 22) de l'implant (20), sont réalisées planes.

5. Instrument selon l'une des revendications précédentes, **caractérisé en ce que** la face supérieure et la face inférieure de l'élément d'espacement (8), tournées vers les parties (21, 22) de l'implant (20), sont légèrement inclinées l'une par rapport à l'autre.

6. Instrument selon l'une des revendications précédentes, **caractérisé en ce que** l'élément d'espacement comprend deux organes d'espacement (8) recevant entre eux les surfaces d'articulation (27, 28).

7. Instrument selon la revendication 6, **caractérisé en ce que** les organes d'espacement s'étendent parallèlement l'un à l'autre.

8. Instrument selon l'une des revendications précédentes, **caractérisé en ce que** le dispositif de retenue (5, 6) et les parties (21, 22) de l'implant (20) présentent, pour réaliser la liaison détachable, des éléments de serrage (12 ; 29) coulissant l'un dans l'autre qui peuvent être serrés l'un contre l'autre transversalement à leur direction d'enfichage.

9. Instrument selon la revendication 8, **caractérisé en ce que** les éléments de serrage (12 ; 29) sont des saillies et retraits qui s'engagent les uns dans les autres.

10. Instrument selon la revendication 9, **caractérisé en ce que** les saillies et retraits sont des goupilles (12) et des perçages (29) recevant celles-ci.

11. Instrument selon la revendication 8 ou 9, **caractérisé en ce que** les éléments de serrage (12, 29) peuvent être serrés les uns contre les autres dans une direction qui s'étend transversalement au déplacement des pièces (21, 22) de l'implant (20) à travers l'élément d'espacement (8) s'engageant entre elles.

12. Instrument selon l'une des revendications 8 à 11, **caractérisé en ce que** les éléments de serrage (12) sur l'instrument (1) peuvent être déplacés transversalement à leur direction d'enfichage.

13. Instrument selon la revendication 12, **caractérisé en ce que** l'instrument (1) présente deux bras (5, 6) pivotables l'un par rapport à l'autre, qui portent chacun à leur extrémité libre des éléments de serrage (12) pour les deux parties (21, 22) de l'implant (20), et **en ce que** sur l'instrument est agencé un dispositif de tension (13) qui permet de faire pivoter les bras (5, 6) l'un par rapport à l'autre.

14. Instrument selon la revendication 13, **caractérisé en ce que** les bras (5, 6) peuvent pivoter élastiquement l'un par rapport à l'autre.

15. Instrument selon la revendication 14, **caractérisé en ce que** le dispositif de tension est une douille (13) coiffant les bras (5, 6) et déplaçable le long des bras (5, 6).

16. Instrument selon la revendication 15, **caractérisé en ce que** la douille (13) peut tourner autour de son axe longitudinal pour se déplacer le long des bras (5, 6) et est montée sur l'instrument (1) via un pas de vis.

17. Instrument selon l'une des revendications 13 à 16, **caractérisé en ce que** chaque bras (5, 6) porte un organe d'espacement (8).

18. Instrument selon l'une des revendications 13 à 17, **caractérisé en ce que** chaque bras (5, 6) porte à son extrémité libre une mâchoire de serrage (7) sur laquelle des éléments de serrage (12) respectifs pour les deux parties (21, 22) de l'implant (20) et un organe d'espacement (8) sont agencés et forment une butée (10) contre laquelle sont en appui les parties (21, 22) de l'implant (20) lorsque l'instrument (1) et l'implant (20) sont reliés.
